# EUROPÄISCHE PATENTSCHRIFT

(11) **EP 3 329 887 B1**
(45) Veröffentlichungstag und Bekanntmachung des Hinweises auf die Patenterteilung: **22.04.2020**
(21) Anmeldenummer: 16201503.6
(22) Anmeldetag: 30.11.2016
(51) Int. Cl.: A61F 9/06, G02B 7/00

(54) **OPTISCHER BLENDSCHUTZFILTER**
OPTICAL GLARE SHIELD FILTER
FILTRE OPTIQUE ANTI-ÉBLOUISSEMENT

(43) Veröffentlichungstag der Anmeldung: 06.06.2018
(73) Patentinhaber: Optrel Holding AG, 9630 Wattwil (CH)
(72) Erfinder: Hofer Kraner, Ramon, 9100 Herisau (CH); Esposito, Martin, 8640 Rapperswil (CH); Heusser, Jonathan, 8713 Uerikon (CH); Sheng, Yang, 9630 Wattwil (CH); Landolt, Marco, 8752 Näfels (CH); Blöchlinger, Daniel, 8735 St. Gallenkappel (CH)
(74) Vertreter: Daub, Thomas

(56) Entgegenhaltungen:
- EP-A1- 2 839 817
- US-A1- 2006 285 330
- US-A1- 2014 168 546
- US-A1- 2014 215 673
- US-A1- 2015 033 430

## Beschreibung

### Stand der Technik

Die Erfindung betrifft einen optischen Blendschutzfilter.

Es ist bereits ein optischer Blendschutzfilter für eine Blendschutzvorrichtung, mit zumindest einer Flüssigkristallzelle, welche zumindest eine Flüssigkristallschicht und zumindest eine erste Elektrodenschichteinheit zu einer Ausrichtung von Kristallmolekülen der zumindest einen Flüssigkristallschicht aufweist, und mit zumindest einem ersten Kontaktelement zu einer elektrischen Kontaktierung der zumindest einen ersten Elektrodenschicht, vorgeschlagen worden.

Die Aufgabe der Erfindung besteht insbesondere darin, eine gattungsgemäße Vorrichtung mit verbesserten Eigenschaften hinsichtlich einer Schaltgeschwindigkeit sowie hinsichtlich einer Homogenität der Schaltgeschwindigkeitsverteilung bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

Ein Optischer Blendschutzfilter gemäß dem Oberbegriff von Anspruch 1 ist aus dem Dokument US-A-2014/0168546 bekannt.

### Vorteile der Erfindung

Die Erfindung geht aus von einem optischen Blendschutzfilter für eine Blendschutzvorrichtung, mit zumindest einer Flüssigkristallzelle, welche zumindest eine Flüssigkristallschicht und zumindest eine erste Elektrodenschichteinheit zu einer Ausrichtung von Kristallmolekülen der zumindest einen Flüssigkristallschicht aufweist, und mit zumindest einem ersten Kontaktelement zu einer elektrischen Kontaktierung der zumindest einen ersten Elektrodenschicht.

Es wird vorgeschlagen, dass der optische Blendschutzfilter zumindest ein zweites Kontaktelement zu einer elektrischen Kontaktierung der zumindest einen ersten Elektrodenschichteinheit aufweist, welche wesentlich von dem ersten Kontaktelement beabstandet ist. Vorzugsweise weist die Flüssigkristallzelle zumindest zwei Elektrodenschichteinheiten auf, welche gemeinsam zu einer Ausrichtung von Kristallmolekülen der zumindest einen Flüssigkristallschicht vorgesehen sind. Bevorzugt sind die Elektrodenschichteinheiten auf gegenüberliegenden Seiten der zumindest einen Flüssigkristallschicht angeordnet. Vorzugsweise sind das erste Kontaktelement und das zweite Kontaktelement an einem Umfang der Elektrodenschichteinheit angeordnet. Unter einem "optischen Blendschutzfilter" soll insbesondere ein optischer Filter für eine Blendschutzvorrichtung verstanden werden, welcher insbesondere ein Schutzglas und/oder ein Kunststoffschutzglas bildet. Vorzugsweise soll darunter insbesondere ein optischer Filter verstanden werden, dessen Lichtdurchlässigkeit einstellbar ausgeführt ist. Bevorzugt soll darunter insbesondere ein optischer Schweißschutzfilter mit einer automatischen Verdunkelung verstanden werden. Besonders bevorzugt weist der Blendschutzfilter zumindest eine in der Transmission schaltbare Flüssigkristallebene auf. Es sind verschiedene, einem Fachmann als sinnvoll erscheinende Ausgestaltungen des optischen Blendschutzfilters denkbar, insbesondere soll darunter jedoch ein ADF, auch "automatic darkening filter" oder "automatischer Schweißerschutzfilter" genannt, verstanden werden. Unter einer "Blendschutzvorrichtung" soll in diesem Zusammenhang insbesondere eine Vorrichtung verstanden werden, welche zu einem Schutz eines Benutzers vor zu großer Helligkeit und/oder Funken vorgesehen ist. Vorzugsweise soll darunter insbesondere eine Vorrichtung verstanden werden, welche zu einem Schutz von Augen und/oder einem Gesichtsbereich eines Benutzers während eines Schweiß- und/oder Schleifprozesses dient. Bevorzugt soll darunter insbesondere eine Vorrichtung verstanden werden, welche insbesondere zu einem Schutz der Augen eines Benutzers zumindest während eines Schweißprozesses dient. Es sind verschiedene, einem Fachmann als sinnvoll erscheinende Ausgestaltungen einer Schutzvorrichtung denkbar, wie beispielsweise als Schweißhelm, -schirm, -maske und/oder -schild. Ferner sind verschiedene, einem Fachmann als sinnvoll erscheinende Flüssigkristallzellen denkbar, wie insbesondere eine TN-Flüssigkristallzelle mit der Twisted-Nematic-Technik. Grundsätzlich wären jedoch auch andere, einem Fachmann als sinnvoll erscheinende Ausbildungen der Flüssigkristallzellen denkbar, wie beispielsweise als STN-Flüssigkristallzellen mit der Super-Twisted-Nematic-Technik, DSTN-Flüssigkristallzellen mit der Double-Super-Twisted-Nematic-Technik, TSTN-Flüssigkristallzellen mit der Triple-Super-Twisted-Nematic-Technik, VA-Flüssigkristallzellen mit der Vertical-Alignment-Technik, insbesondere PVA/MVA-Flüssigkristallzellen mit der Patterned-Vertical-Alignment- und/oder Multi-Domain-Vertical-Alignment-Technik, IPS-Flüssigkristallzellen mit der In-Plane-Switching-Technik, FLCD-Flüssigkristallzellen, also ferroelektrische Flüssigkristallzellen, und/oder TN-Flüssigkristallzellen mit der Guest-Host-Technik.

Unter einer "Elektrodenschichteinheit" soll in diesem Zusammenhang insbesondere eine vorzugsweise dünne, schichtartige Einheit der Flüssigkristallzelle verstanden werden, die zu einer Ausrichtung von Kristallmolekülen der Flüssigkristallzelle vorgesehen ist. Vorzugsweise ist die Elektrodenschichteinheit dazu vorgesehen, bei einer anliegenden Spannung ein elektrisches Feld zu erzeugen, welches die Kristallmoleküle ausrichtet. Bevorzugt wird das elektrische Feld zwischen zwei Elektrodenschichteinheiten der Flüssigkristallzelle erzeugt. Eine Elektrodenschichteinheit kann dabei sowohl aus einer einzelnen Schicht, aus mehreren übereinander angeordneten Schichten und/oder aus mehreren in einer Schichtebene angeordneten Teilschichtsegmenten bestehen. Grundsätzlich sind jedoch auch noch weitere, einem Fachmann als sinnvoll erscheinende Ausgestaltungen der Elektrodenschichteinheit denkbar. Ferner soll in diesem Zusammenhang unter einem "Kontaktelement" insbesondere ein Element verstanden werden, welches zu einer elektrischen Kontaktierung einer Elektrodenschichteinheit vorgesehen ist. Vorzugsweise soll darunter insbesondere ein Element verstanden werden, welches eine Kontaktierstelle der Elektrodenschichteinheit bildet, welche durch eine Dichtung der Flüssigkristallzelle hindurch die Elektrodenschichteinheit elektrisch kontaktiert. Bevorzugt soll darunter insbesondere ein Element verstanden werden, welches die Elektrodenschichteinheit direkt kontaktiert und über welches eine Spannung an die Elektrodenschichteinheit angelegt werden kann. Das Kontaktelement kann dabei sowohl von einem einzelnen Kontaktpunkt als auch von einer Kontaktfläche gebildet sein. Unter "wesentlich beabstandet" soll in diesem Zusammenhang insbesondere verstanden werden, dass ein minimaler Abstand zwischen den Kontaktelementen, insbesondere zwischen den Kontaktpunkten der Kontaktelemente zu der Elektrodenschicht, zumindest 1 cm, vorzugsweise zumindest 2 cm und besonders bevorzugt zumindest 3 cm beträgt. Vorzugsweise soll darunter insbesondere verstanden werden, dass ein minimaler Abstand, insbesondere entlang des Umfangs der Elektrodenschicht, zwischen den Kontaktelementen, insbesondere zwischen den Kontaktpunkten der Kontaktelemente zu der Elektrodenschicht, zumindest 1%, vorzugsweise zumindest 3%, bevorzugt zumindest 5% und besonders bevorzugt zumindest 10% eines Gesamtumfangs der Elektrodenschichteinheit entlang einer Außenkontur beträgt. Der Gesamtumfang der Elektrodenschichteinheit erstreckt sich bevorzugt in einer Haupterstreckungsebene der Elektrodenschichteinheit und bildet insbesondere einen maximalen Umfang der Elektrodenschicht. Dabei soll unter einer "Haupterstreckungsebene" einer Schicht und/oder einer Baueinheit insbesondere eine Ebene verstanden werden, welche parallel zu einer größten Seitenfläche eines kleinsten gedachten Quaders ist, welcher die Schicht und/oder die Baueinheit gerade noch vollständig umschließt, und insbesondere durch den Mittelpunkt des Quaders verläuft. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Durch die erfindungsgemäße Ausgestaltung kann insbesondere ein vorteilhafter Blendschutzfilter bereitgestellt werden. Insbesondere kann ein vorteilhaft schnelles und homogenes Ansprechverhalten des Blendschutzfilters bereitgestellt werden. Durch eine multiple Kontaktierung der zumindest einen ersten Elektrodenschichteinheit ist es möglich, eine vorteilhaft hohe Schaltgeschwindigkeit der Flüssigkristallzelle zu erreichen. Ferner kann eine vorteilhaft homogene Schaltgeschwindigkeitsverteilung der Flüssigkristallzelle erreicht werden.

Ferner wird vorgeschlagen, dass das zumindest eine zweite Kontaktelement auf einer dem zumindest einen ersten Kontaktelement gegenüberliegenden Seite der ersten Elektrodenschichteinheit angeordnet ist. Vorzugsweise ist zumindest ein Teilbereich der ersten Elektrodenschichteinheit zwischen dem ersten Kontaktelement und dem zweiten Kontaktelement angeordnet. Bevorzugt sind das erste Kontaktelement und das zweite Kontaktelement an gegenüberliegenden Punkten eines Gesamtumfangs der Elektrodenschichteinheit angeordnet. Dadurch kann insbesondere ein besonders vorteilhafter Blendschutzfilter bereitgestellt werden. Ferner kann eine vorteilhaft homogene Schaltgeschwindigkeitsverteilung der Flüssigkristallzelle erreicht werden. Durch die multiple Kontaktierung kann ein vorteilhaft gleichmäßiges Aufladen ermöglicht werden. Es kann ein Aufladen der Flüssigkristallzelle aus verschiedenen Richtungen erreicht werden, wodurch die Flüssigkristallzelle vorteilhaft homogen sowie vorteilhaft schnell aufgeladen werden kann. Ein Abdunkeln kann in einer sehr kurzen Zeitspanne, insbesondere ca. 100 µs, erreicht werden. Die vorteilhafte Schaltgeschwindigkeitsverteilung ist insbesondere auch für die Messung der Schaltzeit von Bedeutung, da gemäß der Normvorgaben (EN379) keine Anforderung an den Ort der Messung besteht. Dies heißt, der Prüfer kann den Ort selber bestimmen und kann so einen ungünstigen Ort prüfen. Mit der multiplen Kontaktierung sinkt die Bedeutung des Orts der Messung.

Des Weiteren wird vorgeschlagen, dass der optische Blendschutzfilter zumindest ein weiteres Kontaktelement zu einer elektrischen Kontaktierung der zumindest einen ersten Elektrodenschicht, welche wesentlich von dem ersten Kontaktelement und dem zweiten Kontaktelement beabstandet ist, aufweist. Vorzugsweise ist ein Kontaktpunkt des weiteren Kontaktelements zu der Elektrodenschichteinheit wesentlich zu den Kontaktpunkten des ersten Kontaktelements und des zweiten Kontaktelements zu der Elektrodenschichteinheit beabstandet. Bevorzugt entspricht ein Abstand des ersten Kontaktelements zu dem zweiten Kontaktelement zumindest im Wesentlichen einem Anstand zwischen dem zweiten Kontaktelement zu dem weiteren Kontaktelement. Dadurch kann insbesondere ein besonders vorteilhafter Blendschutzfilter bereitgestellt werden. Vorzugsweise kann durch die multiple Kontaktierung ein vorteilhaft gleichmäßiges Aufladen ermöglicht werden. Durch die hohe Anzahl an Kontaktierungen kann eine vorteilhafte Aufladecharakteristik erreicht werden. Die Aufladung der der Elektroden und der Flüssigkristallschicht und somit der als Kondensator wirkenden Flüssigkristallzelle insgesamt kann durch die multiple Kontaktierung vorteilhaft schnell erfolgen. Vergleicht man die globale Abdunkelung, was ungefähr der Aufladung entspricht, zu einem Zeitpunkt t mit verschiedenen Kontaktierungen, so kann man beobachten, dass die Gesamtabdunkelung mit der Anzahl der Kontaktierungen zunimmt. Die Erhöhung der Abdunkelungs- und/oder der Aufladegeschwindigkeit kann im Bereich von 0,5 - 50% liegen.

Es wird ferner vorgeschlagen, dass die Kontaktelemente zu einer elektrischen Kontaktierung der zumindest einen ersten Elektrodenschichteinheit in Umfangsrichtung um die erste Elektrodenschichteinheit zumindest im Wesentlichen gleichmäßig verteilt sind. Vorzugsweise sind die Kontaktelemente zu einer elektrischen Kontaktierung der zumindest einen ersten Elektrodenschichteinheit in Umfangsrichtung um die erste Elektrodenschichteinheit zumindest im Wesentlichen gleichmäßig entlang eines Umfangs der ersten Elektrodenschichteinheit verteilt. Unter "in Umfangsrichtung um die erste Elektrodenschichteinheit zumindest im Wesentlichen gleichmäßig verteilt" soll in diesem Zusammenhang insbesondere verstanden werden, dass die Abstände zwischen zwei entlang des Umfangs der Elektrodenschichteinheit benachbarten Kontaktelementen entlang des Umfangs der Elektrodenschichteinheit zumindest im Wesentlichen identisch sind. Vorzugsweise soll darunter insbesondere verstanden werden, dass ein kleinster Abstand zwischen zwei entlang des Umfangs der Elektrodenschichteinheit benachbarten Kontaktelementen entlang des Umfangs der Elektrodenschichteinheit zumindest 20%, vorzugsweise zumindest 40% und besonders bevorzugt zumindest 60% eines größten Abstands zwischen zwei entlang des Umfangs der Elektrodenschichteinheit benachbarten Kontaktelementen entlang des Umfangs der Elektrodenschichteinheit beträgt. Dadurch kann eine vorteilhaft homogene Schaltgeschwindigkeitsverteilung der Flüssigkristallzelle erreicht werden. Durch die multiple Kontaktierung kann ein vorteilhaft gleichmäßiges Aufladen ermöglicht werden. Es kann ein Aufladen der Flüssigkristallzelle aus verschiedenen Richtungen erreicht werden, wodurch die Flüssigkristallzelle vorteilhaft homogen sowie vorteilhaft schnell aufgeladen werden kann.

Es wird weiter vorgeschlagen, dass der optische Blendschutzfilter einen Nasenausschnitt aufweist, welcher zu einer zumindest teilweisen Aufnahme einer Nase eines Benutzers vorgesehen ist. Unter einem "Nasenausschnitt" soll in diesem Zusammenhang insbesondere eine immaterielle Aussparung in einem zumindest teilweise transluzenten Teilbereich des optischen Blendschutzfilters verstanden werden, welcher in zumindest einer Betriebsstellung der Blendschutzvorrichtung zumindest teilweise zu einer zumindest teilweisen Aufnahme einer Nase eines Benutzers vorgesehen ist. Vorzugsweise ist die Aussparung in jedem Punkt in zumindest einer Ebene, insbesondere parallel zu einer Haupterstreckungsebene des Blendschutzfilters, in einem Winkelbereich von zumindest 180° von einem materiellen Teilbereich, insbesondere einem zumindest teilweise transluzenten Teilbereich, des optischen Blendschutzfilters umgeben. Vorzugsweise umgreift der optische Blendschutzfilter in zumindest einer Betriebsstellung der Schutzvorrichtung eine Nase des Benutzers. Besonders bevorzugt beträgt eine vertikale Erstreckung des Nasenausschnitts zumindest 10%, vorzugsweise zumindest 30%, bevorzugt zumindest 50% und besonders bevorzugt zumindest 55% einer vertikalen Erstreckung des optischen Blendschutzfilters. Dadurch kann insbesondere ein vorteilhaft hoher Komfort des optischen Blendschutzfilters erreicht werden. Durch die multiple Kontaktierung kann trotz des Nasenausschnitts eine vorteilhaft homogene Schaltgeschwindigkeitsverteilung sowie eine schnelle Abdunkelung der Flüssigkristallzelle erreicht werden.

Ferner wird vorgeschlagen, dass der optische Blendschutzfilter zumindest zwei weitere Kontaktelemente aufweist, welche jeweils eine zweite Elektrodenschichteinheit der zumindest einen Flüssigkristallzelle elektrisch kontaktieren. Vorzugsweise weist die Flüssigkristallzelle eine zweite Elektrodenschichteinheit auf, welche sich parallel zu der ersten Elektrodenschichteinheit erstreckt. Die zweite Elektrodenschichteinheit ist vorzugsweise auf einer der ersten Elektrodenschichteinheit gegenüberliegenden Seite der Flüssigkristallschicht angeordnet. Bevorzugt ist die zweite Elektrodenschichteinheit mit zu den Kontaktelementen, welche die erste Elektrodenschichteinheit elektrisch kontaktieren, korrespondierenden Kontaktelementen verbunden. Die Kontaktelemente, welche die zweite Elektrodenschichteinheit elektrisch kontaktieren, sind ebenfalls wesentlich zueinander beabstandet angeordnet. Die Kontaktelemente, welche die zweite Elektrodenschichteinheit elektrisch kontaktieren, weisen gegenüber der zweiten Elektrodenschichteinheit die gleiche Anordnung auf wie Kontaktelemente, welche die erste Elektrodenschichteinheit elektrisch kontaktieren, gegenüber der ersten Elektrodenschicht. Dadurch kann ein vorteilhafter Blendschutzfilter bereitgestellt werden. Insbesondere kann ein vorteilhaft schnelles und homogenes Ansprechverhalten des Blendschutzfilters bereitgestellt werden. Durch eine multiple Kontaktierung der zumindest einen ersten Elektrodenschichteinheit ist es möglich, eine vorteilhaft hohe Schaltgeschwindigkeit der Flüssigkristallzelle zu erreichen. Ferner kann eine vorteilhaft homogene Schaltgeschwindigkeitsverteilung der Flüssigkristallzelle erreicht werden.

Die Anordnung der Kontaktelemente, welche die zweite Elektrodenschichteinheit elektrisch kontaktieren, können sowohl kongruent als auch abweichend zur Anordnung der Kontaktelemente, welche die ersten Elektrodenschichteinheit elektrisch kontaktieren, ausgeführt sein.

Des Weiteren wird eine Blendschutzvorrichtung mit dem zumindest einen optischen Blendschutzfilter und mit zumindest einer Steuer- und/oder Regeleinheit vorgeschlagen, welche dazu vorgesehen ist, abhängig von einem erfassten Arbeitszustand und/oder einer Lichteinstrahlung, eine Durchlässigkeit des optischen Blendschutzfilters zu steuern und/oder zu regeln. Unter einer "Steuer- und/oder Regeleinheit" soll in diesem Zusammenhang insbesondere eine Einheit mit zumindest einer Steuerelektronik verstanden werden. Unter einer "Steuerelektronik" soll insbesondere eine Einheit mit zumindest einer elektronischen Schaltung verstanden werden, welche vorzugsweise aus Spannungs- und Vergleichsregelbausteinen besteht. Grundsätzlich kann die Steuerelektronik jedoch auch komplexer aufgebaut sein, wie insbesondere durch die Nutzung einer anwendungsspezifischen integrierten Schaltung (ASIC) und/oder eines Mikrokontrollerbausteins. Dadurch kann insbesondere eine vorteilhafte Blendschutzvorrichtung bereitgestellt werden. Es kann insbesondere eine Blendschutzvorrichtung mit einem vorteilhaft schnell und homogen aufladenden optischen Blendschutzfilter bereitgestellt werden.

Es wird ferner vorgeschlagen, dass die zumindest einer Steuer- und/oder Regeleinheit den optischen Blendschutzfilter zumindest im Wesentlichen gleichzeitig über das zumindest eine erste Kontaktelement und das eine zweite Kontaktelement ansteuert. Vorzugsweise wird der optische Blendschutzfilter mittels der Steuer- und/oder Regeleinheit gleichzeitig über alle Kontaktelemente angesteuert. Dadurch kann ein vorteilhaft schnelles Aufladen des optischen Blendschutzfilters erreicht werden. Grundsätzlich wäre jedoch auch denkbar, dass die Steuer- und/oder Regeleinheit die Kontaktelemente beispielsweise abhängig von einer Richtung eines Strahlungseingangs ansteuert. Beispielsweise wäre denkbar, dass der optische Blendschutzfilter bei einem Strahlungseingang von der Seite von dieser Seite aus den optischen Blendschutzfilter abdunkelt.

Es wird weiter vorgeschlagen, dass die Blendschutzvorrichtung zumindest eine Schildeinheit aufweist, in welcher der zumindest eine optische Blendschutzfilter fest aufgenommen ist. Unter einer "Schildeinheit" soll in diesem Zusammenhang insbesondere eine Einheit verstanden werden, welche in einer regulären Betriebsstellung vor einem Gesicht des Benutzers angeordnet ist. Vorzugsweise soll darunter insbesondere eine Einheit verstanden werden, welche in einer Betriebsstellung der Blendschutzvorrichtung insbesondere zumindest einen wesentlichen Teil eines Gesichts eines Benutzers verdeckt. Vorzugsweise soll darunter insbesondere eine Einheit verstanden werden, welche zu einem Schutz eines Gesichts, wie beispielsweise vor umherfliegenden Funken, vorgesehen ist. Bevorzugt ist die Schildeinheit insbesondere dazu vorgesehen, eine Schutzbarriere zwischen einem Arbeitsbereich und einem Gesicht des Benutzers zu bilden. Dadurch kann eine vorteilhafte Blendschutzvorrichtung bereitgestellt werden. Es kann eine vorteilhaft sichere Blendschutzvorrichtung bereitgestellt werden.

Der erfindungsgemäße optische Blendschutzfilter sowie die Blendschutzvorrichtung sollen hierbei nicht auf die oben beschriebene Anwendung und Ausführungsform beschränkt sein. Insbesondere können der erfindungsgemäße optische Blendschutzfilter sowie die Blendschutzvorrichtung zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen sind fünf Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine Blendschutzvorrichtung mit einer Schildeinheit, mit einem erfindungsgemäßen optischen Blendschutzfilter und mit einer Vorsatzscheibe auf einem Kopf eines Benutzers in einer schematischen Darstellung,
- Fig. 2: den erfindungsgemäßen optischen Blendschutzfilter mit einer Flüssigkristallzelle und mit einer Kontakteinheit in einer schematischen Darstellung,
- Fig. 3: den erfindungsgemäßen optischen Blendschutzfilter mit der Flüssigkristallzelle und mit der Kontakteinheit, welche mehrere Kontaktelemente aufweist, in einer schematischen Frontalansicht,
- Fig. 4: den erfindungsgemäßen optischen Blendschutzfilter mit der Flüssigkristallzelle und mit der Kontakteinheit in einer schematischen Seitenansicht,
- Fig. 5: einen Teilausschnitt V des erfindungsgemäßen optischen Blendschutzfilters mit der Flüssigkristallzelle und mit der Kontakteinheit in einer schematischen Seitenansicht,
- Fig. 6: einen Teilausschnitt VI der Flüssigkristallzelle des erfindungsgemäßen optischen Blendschutzfilters in einer schematischen Schnittdarstellung,
- Fig. 7: den erfindungsgemäßen optischen Blendschutzfilter während eines Abdunklungsvorgangs zu einem Zeitpunkt t in einer schematischen Frontalansicht,
- Fig. 8: einen optischen Blendschutzfilter mit lediglich einem Kontaktelement während eines Abdunklungsvorgangs zu einem Zeitpunkt t in einer schematischen Frontalansicht,
- Fig. 9: einen alternativen erfindungsgemäßen optischen Blendschutzfilter mit einer Flüssigkristallzelle und mit einer Kontakteinheit in einer schematischen Explosionsdarstellung,
- Fig. 10: den alternativen erfindungsgemäßen optischen Blendschutzfilter während eines Abdunklungsvorgangs zu einem Zeitpunkt t in einer schematischen Frontalansicht,
- Fig. 11: einen optischen Blendschutzfilter mit lediglich einem Kontaktelement während eines Abdunklungsvorgangs zu einem Zeitpunkt t in einer schematischen Frontalansicht,
- Fig. 12: einen weiteren alternativen erfindungsgemäßen optischen Blendschutzfilter während eines Abdunklungsvorgangs zu einem Zeitpunkt t in einer schematischen Frontalansicht,
- Fig. 13: einen weiteren alternativen erfindungsgemäßen optischen Blendschutzfilter während eines Abdunklungsvorgangs zu einem Zeitpunkt t in einer schematischen Frontalansicht und
- Fig. 14: einen weiteren alternativen erfindungsgemäßen optischen Blendschutzfilter während eines Abdunklungsvorgangs zu einem Zeitpunkt t in einer schematischen Frontalansicht.

### Beschreibung der Ausführungsbeispiele

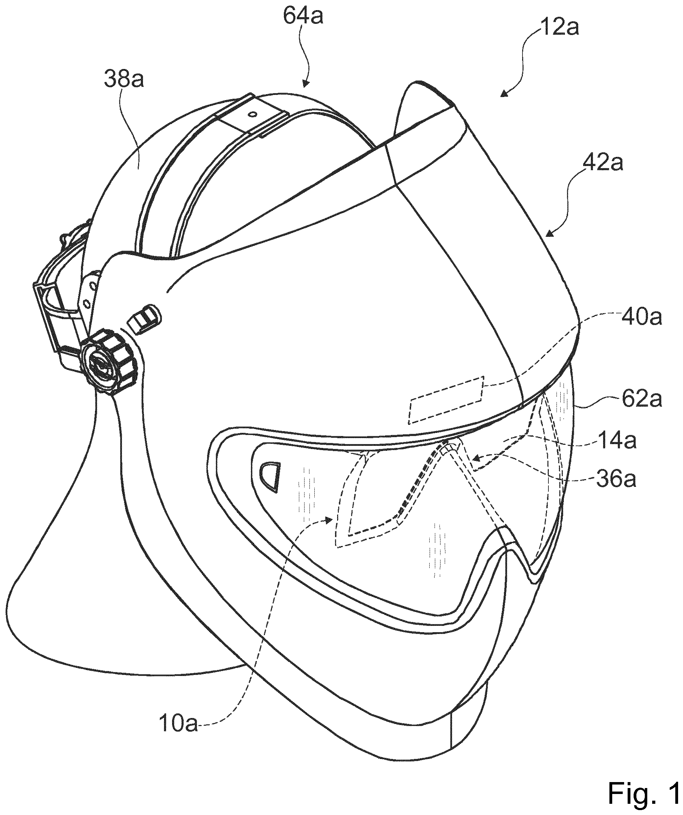

Die Figur 1 zeigt eine Blendschutzvorrichtung 12a auf einem Kopf eines Benutzers 38a. Die Blendschutzvorrichtung 12a ist dazu vorgesehen, während eines Betriebs von einem Benutzer 38a auf dem Kopf getragen zu werden. Figur 1 zeigt die Blendschutzvorrichtung 12a in einer Betriebsstellung. Diese ist von einem Schweißhelm gebildet. Grundsätzlich wäre jedoch auch eine andere, einem Fachmann als sinnvoll erscheinende Ausbildung der Blendschutzvorrichtung 12a denkbar.

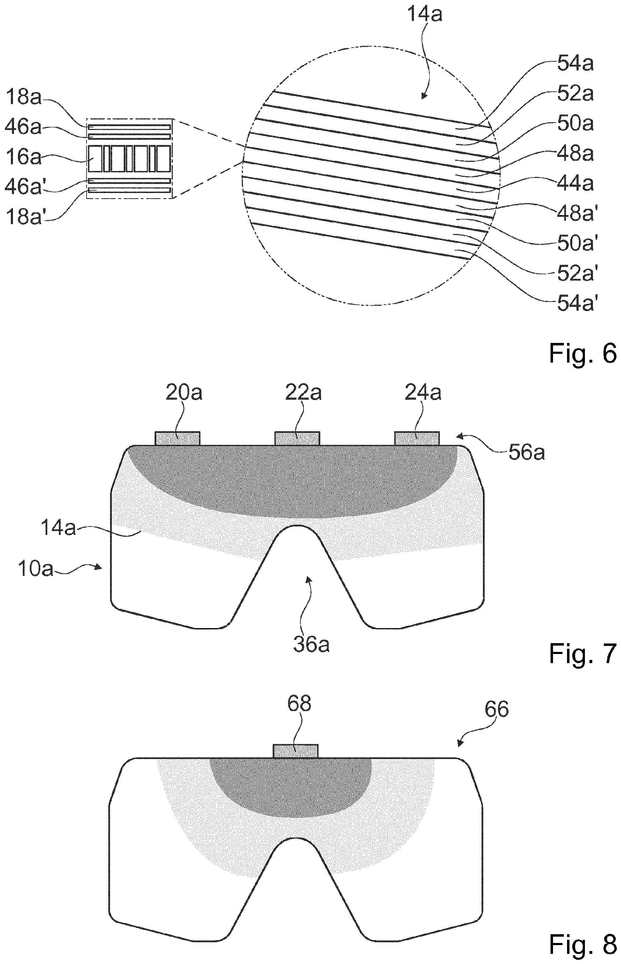

Die Blendschutzvorrichtung 12a weist einen optischen Blendschutzfilter 10a auf. Der optische Blendschutzfilter 10a ist von einem elektrooptischen Filter gebildet. Der optische Blendschutzfilter 10a ist von einem automatic darkening filter, kurz ADF, gebildet. Der optische Blendschutzfilter 10a weist eine Flüssigkristallzelle 14a auf. Die Flüssigkristallzelle 14a besteht aus mehreren Schichten. Die Flüssigkristallzelle 14a ist als ein Mehrschichtverbund ausgebildet. Eine Anzahl von Schichten ist hierbei lediglich beispielhaft und kann grundsätzlich variieren. Die Flüssigkristallzelle 14a ist von einer TN-Flüssigkristallzelle gebildet. Die Flüssigkristallzelle 14a basiert daher auf der Twisted-Nematic-Technik. Grundsätzlich wären jedoch auch andere, einem Fachmann als sinnvoll erscheinende Ausbildungen der Flüssigkristallzelle 14a denkbar, wie beispielsweise als STN-Flüssigkristallzelle mit der Super-Twisted-Nematic-Technik, DSTN-Flüssigkristallzelle mit der Double-Super-Twisted-Nematic-Technik, TSTN-Flüssigkristallzelle mit der Triple-Super-Twisted-Nematic-Technik, VA-Flüssigkristallzelle mit der Vertical-Alignment-Technik, insbesondere PVA/MVA-Flüssigkristallzelle mit der Patterned-Vertical-Alignment- und/oder Multi-Domain-Vertical-Alignment-Technik, IPS-Flüssigkristallzelle mit der In-Plane-Switching-Technik, FLCD-Flüssigkristallzelle, also ferroelektrische Flüssigkristallzelle, und/oder TN-Flüssigkristallzelle mit der Guest-Host-Technik. Die Flüssigkristallzelle 14a weist eine Flüssigkristallschicht 16a auf. Die Flüssigkristallzelle 14a weist eine Flüssigkristallebene 44a auf. Die Flüssigkristallebene 44a ist von einer transluzenten Flüssigkristallebene gebildet. Die Flüssigkristallebene 44a weist die Flüssigkristallschicht 16a auf. In der Flüssigkristallschicht 16a befindet sich eine Vielzahl von Kristallmolekülen sowie Abstandshalter. Auf beiden Seiten der Flüssigkristallschicht 16a ist jeweils eine Polyimid-Schicht 46a, 46a' angeordnet. Grundsätzlich wäre jedoch auch eine andere, einem Fachmann als sinnvoll erscheinende Schicht zu einer Ausrichtung der Moleküle denkbar. Ferner weist die Flüssigkristallzelle 14a eine erste Elektrodenschichteinheit 18a zu einer Ausrichtung von Kristallmolekülen der Flüssigkristallschicht 16a auf. Des Weiteren weist die Flüssigkristallzelle 14a eine zweite Elektrodenschichteinheit 18a' zu einer Ausrichtung von Kristallmolekülen der Flüssigkristallschicht 16a auf. Die Elektrodenschichteinheiten 18a, 18a' dienen gemeinsam zu einer Ausrichtung von Kristallmolekülen der Flüssigkristallschicht 16a. Die Elektrodenschichteinheiten 18a, 18a' sind jeweils auf den der Flüssigkristallschicht 16a abgewandten Seiten der Polyimid-Schichten 46a, 46a' angeordnet. Die Elektrodenschichteinheiten 18a, 18a' sind jeweils von einer einzelnen Schicht gebildet. Grundsätzlich wäre jedoch auch denkbar, dass die Elektrodenschichteinheiten 18a, 18a' jeweils aus mehreren übereinander angeordneten Schichten und/oder aus mehreren in einer Schichtebene angeordneten Teilschichtsegmenten bestehen. Die Elektrodenschichteinheiten 18a, 18a' sind jeweils von einer transparenten Indiumzinnoxid-Schicht gebildet. Grundsätzlich wäre jedoch auch eine andere, einem Fachmann als sinnvoll erscheinende Ausbildung denkbar, wie beispielsweise als Aluminiumzinkoxid-Schicht oder als eine Schicht aus einem vergleichbaren, transluzenten, elektrisch leitenden Material. Ferner befindet sich auf beiden Seiten der Flüssigkristallebene 44a der Flüssigkristallzelle 14a jeweils eine Polarisationsschicht 48a, 48a'. Die Polarisationsschichten 48a, 48a' dienen jeweils zu einer Polarisation von einfallendem Licht. Auf den der Flüssigkristallebene 44a abgewandten Seiten der Polarisationsschichten 48a, 48a' ist jeweils eine Scheibe 50a, 50a' angeordnet. Die Scheiben 50a, 50a' bestehen aus Kunststoff oder Glas. Die Scheiben 50a, 50a' bestehen aus Polykarbonat. Optional ist auf einer Außenseite der Scheiben 50a, 50a' jeweils eine Antireflexionsschicht 52a, 52a' sowie eine Hartschicht 54a, 54a' aufgebracht (Fig. 6).

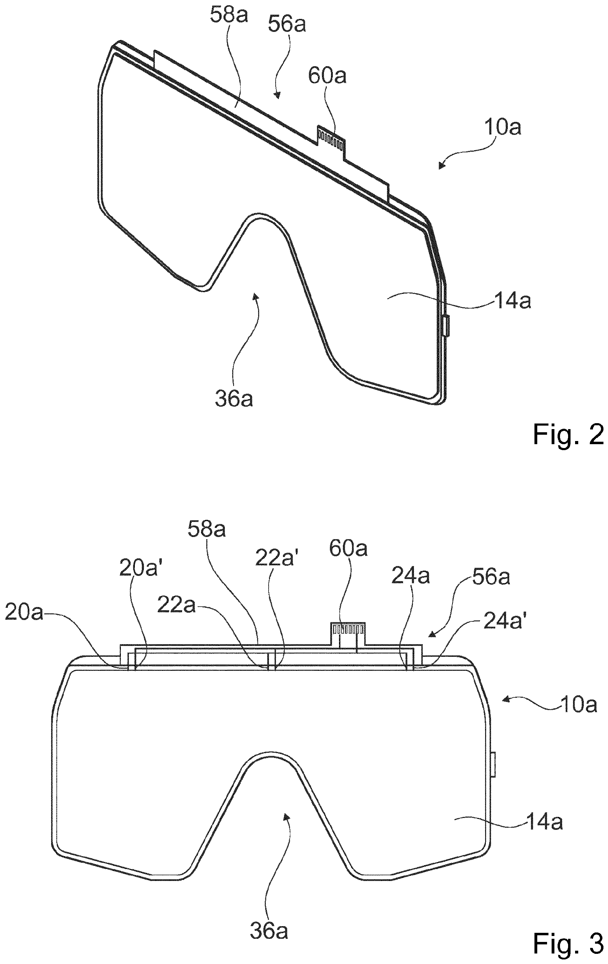

Ferner weist der optische Blendschutzfilter 10a ein erstes Kontaktelement 20a auf. Das erste Kontaktelement 20a ist zu einer elektrischen Kontaktierung der ersten Elektrodenschichteinheit 18a vorgesehen. Der optische Blendschutzfilter 10a weist ferner ein zweites Kontaktelement 22a zu einer elektrischen Kontaktierung der ersten Elektrodenschichteinheit 18a auf. Das zweite Kontaktelement 22a ist wesentlich von dem ersten Kontaktelement 20a beabstandet. Ein Kontaktpunkt des zweiten Kontaktelements 22a zu der ersten Elektrodenschichteinheit 18a ist wesentlich zu dem Kontaktpunkt des ersten Kontaktelements 20a zu der ersten Elektrodenschichteinheit 18a beabstandet. Des Weiteren weist der optische Blendschutzfilter 10a ein weiteres Kontaktelement 24a auf. Das weitere Kontaktelement 24a ist ebenfalls zu einer elektrischen Kontaktierung der ersten Elektrodenschichteinheit 18a vorgesehen. Das weitere Kontaktelement 24a ist von dem ersten Kontaktelement 20a und dem zweiten Kontaktelement 22a wesentlich beabstandet. Ein Kontaktpunkt des weiteren Kontaktelements 24a zu der ersten Elektrodenschichteinheit 18a ist zu den Kontaktpunkten des ersten Kontaktelements 20a und des zweiten Kontaktelements 22a zu der ersten Elektrodenschichteinheit 18a wesentlich beabstandet. Ein Abstand des ersten Kontaktelements 20a zu dem zweiten Kontaktelement 22a entspricht im Wesentlichen einem Abstand des zweiten Kontaktelements 22a zu dem weiteren Kontaktelement 24a. Die Kontaktelemente 20a, 22a, 24a sind nebeneinander angeordnet. Die Kontaktelemente 20a, 22a, 24a sind jeweils an einem Umfang der ersten Elektrodenschichteinheit 18a angeordnet. Die erste Elektrodenschichteinheit 18a ist an den Positionen der Kontaktelemente 20a, 22a, 24a durch eine nicht weiter sichtbare Dichtung der Flüssigkristallzelle 14a nach außen geführt, wo die Kontaktelemente 20a, 22a, 24a die Elektrodenschichteinheit 18a mit einem Flexprint kontaktieren. Elektrodenschichteinheit Die Kontaktelemente 20a, 22a, 24a bilden jeweils eine Kontaktierung der ersten Elektrodenschichteinheit 18a. Der optische Blendschutzfilter 10a weist ferner eine Kontakteinheit 56a auf. Die Kontakteinheit 56a weist die Kontaktelemente 20a, 22a, 24a auf. Die Kontaktelemente 20a, 22a, 24a sind auf einer gemeinsamen flexiblen Leiterplatte 58a der Kontakteinheit 56a aufgebracht. Ferner bildet die Kontakteinheit 56a einen Kontaktstecker 60a aus. Über den Kontaktstecker 60a kann der optische Blendschutzfilter 10a elektrisch mit einer Steuer- und Regeleinheit 40a der Blendschutzvorrichtung 12a verbunden werden. Grundsätzlich kann eine elektrische Verbindung jedoch beispielsweise auch über eine Lötfläche erfolgen. Ferner weist der optische Blendschutzfilter 10a drei weitere Kontaktelemente 20a', 22a', 24a', welche jeweils die zweite Elektrodenschichteinheit 18a' der Flüssigkristallzelle 14a elektrisch kontaktieren, auf. Die zweite Elektrodenschichteinheit 18a' ist mit zu den Kontaktelementen 20a, 22a, 24a, welche die ersten Elektrodenschichteinheit 18a elektrisch kontaktieren, korrespondierenden Kontaktelementen 20a', 22a', 24a' verbunden. Die Kontaktelemente 20a', 22a', 24a', welche die zweite Elektrodenschichteinheit 18a' elektrisch kontaktieren, sind zueinander ebenfalls wesentlich beabstandet angeordnet. Die Kontaktelemente 20a', 22a', 24a', welche die zweite Elektrodenschichteinheit 18a' elektrisch kontaktieren, weisen gegenüber der zweiten Elektrodenschichteinheit 18a' die gleiche Anordnung auf wie Kontaktelemente 20a, 22a, 24a, welche die erste Elektrodenschichteinheit 18a elektrisch kontaktieren, gegenüber der ersten Elektrodenschichteinheit 18a. Die Kontakteinheit 56a weist die Kontaktelemente 20a', 22a', 24a' auf. Die Kontaktelemente 20a', 22a', 24a' sind auf einer den Kontaktelementen 20a, 22a, 24a abgewandten Seite der flexiblen Leiterplatte 58a der Kontakteinheit 56a aufgebracht (Fig. 2, 3).

Grundsätzlich wäre jedoch auch denkbar, dass jedes der Kontaktelemente 20a, 20a', 22a, 22a', 24a, 24a' einzeln, beispielsweise mittels einer Drahtverbindung, mit einem Ansteuerpunkt an den Elektrodenschichteinheiten 18a, 18a' verbunden wird.

Des Weiteren weist der optische Blendschutzfilter 10a einen Nasenausschnitt 36a auf. Der Nasenausschnitt 36a ist von einer immateriellen Aussparung in einem materiellen Teil des optischen Blendschutzfilters 10a gebildet. Der Nasenausschnitt 36a ist von einer immateriellen Aussparung in einem materiellen, zumindest teilweise transluzenten Teilbereich des optischen Blendschutzfilters 10a gebildet. Der Nasenausschnitt 36a ist zu einer teilweisen Aufnahme einer Nase eines Benutzers 38a vorgesehen. Der Nasenausschnitt 36a ist in einer Betriebsstellung der Blendschutzvorrichtung 12a zu einer teilweisen Aufnahme einer Nase des Benutzers 38a vorgesehen. Der optische Blendschutzfilter 10a umgreift in einer Betriebsstellung im Bereich des Nasenausschnitts 36a teilweise die Nase des Benutzers 38a. Der Blendschutzfilter 10a weist eine im Wesentlichen rechteckige Grundform auf, wobei der Nasenausschnitt 36a in die rechteckige Grundform ragt. Der Nasenausschnitt 36a weist eine im Wesentlichen dreieckige Form auf. Die beiden durch den materiellen Teil des optischen Blendschutzfilters 10a begrenzten Seiten weisen einen Winkel von annähernd 54° zueinander auf. Der Nasenausschnitt 36a erstreckt sich von einer unteren Kante, insbesondere von einer Mitte der unteren Kante, des Blendschutzfilters 10a in Richtung eines geometrischen Mittelpunkts des optischen Blendschutzfilters 10a. Der Nasenausschnitt 36a ist nach unten hin nicht von dem Blendschutzfilter 10a begrenzt. Eine Form des Nasenausschnitts 36a ist an eine Form einer Nase angepasst. Eine vertikale Erstreckung des Nasenausschnitts 36a beträgt zumindest 45%, vorzugsweise zumindest 50% und besonders bevorzugt zumindest 55% einer vertikalen Erstreckung des Blendschutzfilters 10a. Die vertikale Erstreckung des Nasenausschnitts 36a beträgt annähernd 57% einer vertikalen Erstreckung des Blendschutzfilters 10a.

Die Blendschutzvorrichtung 12a weist ferner die Steuer- und Regeleinheit 40a auf. Die Steuer- und Regeleinheit 40a ist dazu vorgesehen, abhängig von einem erfassten Arbeitszustand und einer Lichteinstrahlung eine Durchlässigkeit des optischen Blendschutzfilters 10a zu steuern. Die Steuer- und Regeleinheit 40a ist dazu mit einer nicht weiter sichtbaren Sensoreinheit verbunden. Die Sensoreinheit weist einen Sensor auf, der dazu vorgesehen ist, einen Schweißvorgang oder das Auftreten eines hellen Lichts, welches die Augen eines Benutzers 38a schädigen oder auf andere Weise beeinflussen könnte, zu detektieren. Der Sensor der Sensoreinheit ist von einer Fotozelle gebildet. Grundsätzlich wäre jedoch auch eine andere, einem Fachmann als sinnvoll erscheinende Ausbildung des Sensors der Sensoreinheit denkbar. Ferner ist die Steuer- und Regeleinheit 40a nicht weiter sichtbar mit dem optischen Blendschutzfilter 10a verbunden. Die Steuer- und Regeleinheit 40a ist nicht weiter sichtbar über den Kontaktstecker 60a der Kontakteinheit 56a mit dem optischen Blendschutzfilter 10a verbunden. Die Steuer- und Regeleinheit 40a steuert den optischen Blendschutzfilter 10a im Wesentlichen gleichzeitig über das eine erste Kontaktelement 20a und das eine zweite Kontaktelement 22a an. Die Steuer- und Regeleinheit 40a steuert den optischen Blendschutzfilter 10a im Wesentlichen gleichzeitig über die Kontaktelemente 20a, 20a', 22a, 22a', 24a, 24a' an. Die Flüssigkristallschicht 16a des optischen Blendschutzfilters 10a wird dadurch vorteilhaft homogen von der Steuer- und Regeleinheit 40a aktiviert, wenn über die Sensoreinheit ein Schweißverfahren oder ein Lichtblitz erfasst wird. Die Flüssigkristallschicht 16a des optischen Blendschutzfilters 10a wird von der Steuer- und Regeleinheit 40a abgedunkelt, wenn über die Sensoreinheit ein Schweißverfahren oder ein Lichtblitz erfasst wird. Die Flüssigkristallschicht 16a des optischen Blendschutzfilters 10a verringert die Durchlässigkeit von sichtbarem Licht durch den optischen Blendschutzfilter 10a.

Ferner weist die Blendschutzvorrichtung 12a eine Schildeinheit 42a auf. Der optische Blendschutzfilter 10a ist fest in der Schildeinheit 42a aufgenommen. Der optische Blendschutzfilter 10a ist positionsfest in der Schildeinheit 42a aufgenommen. Der Blendschutzfilter 10a ist in einer Ausnehmung in der Schildeinheit 42a eingepasst. Die Schildeinheit 42a besteht aus einem im Wesentlichen formfesten Material. Die Schildeinheit 42a besteht aus einem Kunststoff, welcher insbesondere beständig gegenüber Funken und/oder anderen, beim Schweißen auftretenden Einflüssen ist. Die Schildeinheit 42a ist dazu vorgesehen, insbesondere nach den einschlägigen Normen für Schweißmasken, ein Gesicht und/oder Kopf des Benutzers 38 zu bedecken und zu schützen. Die Schildeinheit 42a weist eine teilweise einer Kopfform angepasste Form auf. Die Schildeinheit 42a ist in einem getragenen Zustand der Blendschutzvorrichtung 12a teilweise um ein Gesicht des Benutzers 38a gebogen (Figur 1).

Die Blendschutzvorrichtung 12a weist ferner eine Vorsatzscheibe 62a auf. Die Vorsatzscheibe 62a ist mit der Schildeinheit 42a über nicht weiter sichtbare Rastelemente verbunden. Vorzugsweise weist die Vorsatzscheibe 62a zwei gegenüberliegende Rastausnehmungen auf, in welche jeweils ein Rastelement der Schildeinheit 42a rastend eingreift. Durch die Verrastung kann die Vorsatzscheibe 62a einfach demontiert werden. Hierdurch sind eine einfache Reinigung und/oder ein einfacher Austausch möglich. Die Vorsatzscheibe 62a ist transparent ausgebildet. Die Vorsatzscheibe 62a ist zu einem Schutz des optischen Blendschutzfilters 10a vorgesehen. Die Vorsatzscheibe 62a verdeckt den optischen Blendschutzfilter 10a von außen.

Des Weiteren weist die Blendschutzvorrichtung 12a eine Kopfbefestigungseinheit 64a auf. Die Kopfbefestigungseinheit 64a ist zu einer Befestigung an dem Kopf des Benutzers 38a vorgesehen. Die Kopfbefestigungseinheit 64a ist von einem Kopfband gebildet. Die Kopfbefestigungseinheit 64a ist nicht weiter sichtbar mit der Schildeinheit 42a verbunden.

Figur 7 zeigt den optischen Blendschutzfilter 10a während eines Abdunklungsvorgangs zu einem Zeitpunkt t. Durch die multiple Kontaktierung der Elektrodenschichteinheiten 18a, 18a', ist es möglich, eine vorteilhaft hohe Schaltgeschwindigkeit der Flüssigkristallzelle 14a zu erreichen. Ferner kann eine vorteilhaft homogene Schaltgeschwindigkeitsverteilung der Flüssigkristallzelle 14a erreicht werden. Im Gegensatz zu einem nicht erfindungsgemäßen optischen Blendschutzfilter 66, bei welchem die Elektrodenschichteinheiten mit jeweils nur einem Kontaktelement 68 elektrisch kontaktiert werden, wie dies in Figur 8 dargestellt ist, kann zu dem selben Zeitpunkt t des Abdunklungsvorgangs mittels dem erfindungsgemäßen optischen Blendschutzfilter 10a eine wesentlich homogenere Schaltgeschwindigkeitsverteilung der Flüssigkristallzelle 14a sowie eine stärkere Abdunkelung der Flüssigkristallzelle 14a erreicht werden. Die Figuren 7 und 8 zeigen einen schematischen Zustand der Abdunkelung der Flüssigkristallzelle 14a bzw. der Flüssigkristallzelle des nicht erfindungsgemäßen optischen Blendschutzfilters 66 zu einem definierten Zeitpunkt t des Abdunklungsvorgangs. Weiße Flächen stellen dabei Teile der Flüssigkristallzelle 14a dar, in welchen noch keine vollständige Ausrichtung der Kristallmoleküle der Flüssigkristallschicht 16a erfolgt ist. Hellgraue Flächen stellen dabei Teile der Flüssigkristallzelle 14a dar, in welchen aktuell eine teilweise Ausrichtung der Kristallmoleküle der Flüssigkristallschicht 16a erfolgt. Dunkelgraue Flächen stellen dabei Teile der Flüssigkristallzelle 14a dar, in welchen eine komplette Ausrichtung der Kristallmoleküle der Flüssigkristallschicht 16a bereits erfolgt ist. Die in den Figuren dargestellten Übergänge zwischen den einzelnen Bereichen sind dabei lediglich der Einfachheit halber diskret dargestellt. Bei einer realen Anwendung sind die Übergänge grundsätzlich graduell.

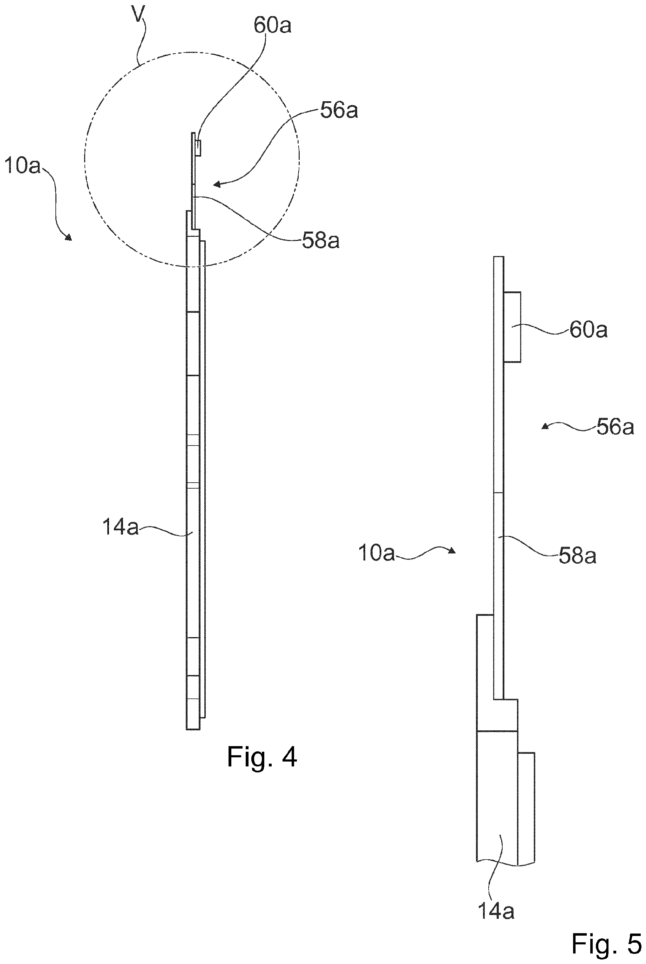

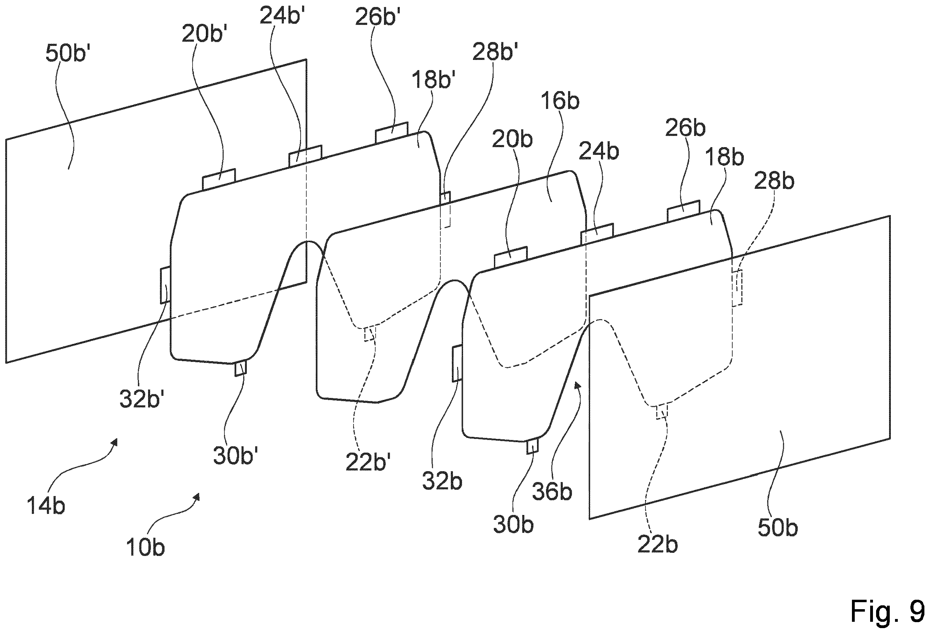

In den Figuren 9 bis 14 sind vier weitere Ausführungsbeispiele der Erfindung gezeigt. Die nachfolgenden Beschreibungen beschränken sich im Wesentlichen auf die Unterschiede zwischen den Ausführungsbeispielen, wobei bezüglich gleichbleibender Bauteile, Merkmale und Funktionen auf die Beschreibung der anderen Ausführungsbeispiele, insbesondere der Figuren 1 bis 8, verwiesen werden kann. Zur Unterscheidung der Ausführungsbeispiele ist der Buchstabe a in den Bezugszeichen des Ausführungsbeispiels der Figuren 1 bis 8 durch die Buchstaben b bis e in den Bezugszeichen der Ausführungsbeispiele der Figuren 9 bis 14 ersetzt. Bezüglich gleich bezeichneter Bauteile, insbesondere in Bezug auf Bauteile mit gleichen Bezugszeichen, kann grundsätzlich auch auf die Zeichnungen und/oder die Beschreibung der anderen Ausführungsbeispiele, insbesondere der Figuren 1 bis 8, verwiesen werden.

Figur 9 zeigt einen alternativen optischen Blendschutzfilter 10b mit einer Flüssigkristallzelle 14b und mit einer Kontakteinheit 56b.

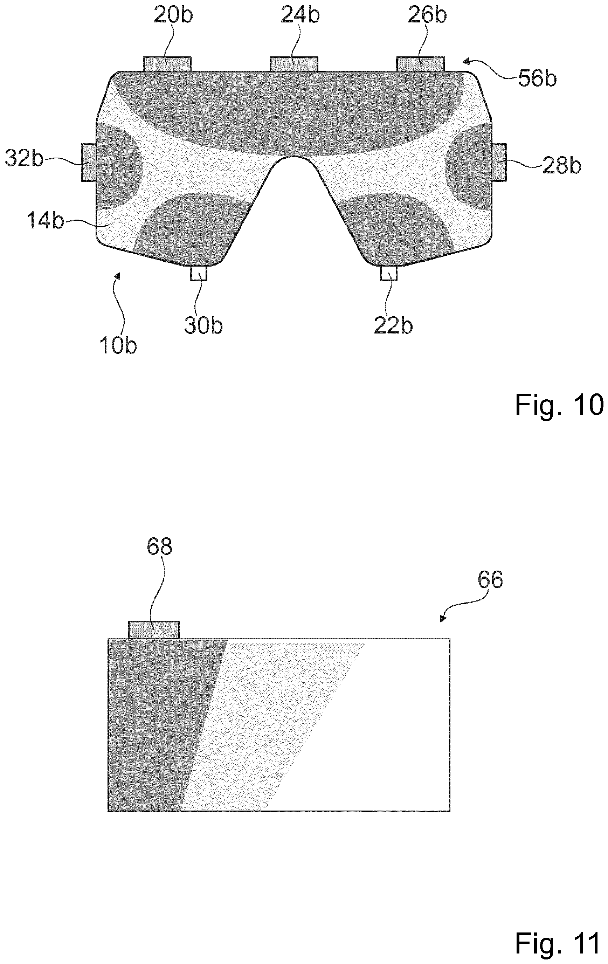

Ferner weist der optische Blendschutzfilter 10b ein erstes Kontaktelement 20b auf. Das erste Kontaktelement 20b ist zu einer elektrischen Kontaktierung der ersten Elektrodenschichteinheit 18b vorgesehen. Der optische Blendschutzfilter 10b weist ferner ein zweites Kontaktelement 22b zu einer elektrischen Kontaktierung der ersten Elektrodenschichteinheit 18b auf. Das zweite Kontaktelement 22b ist von dem ersten Kontaktelement 20b wesentlich beabstandet. Ein Kontaktpunkt des zweiten Kontaktelements 22b zu der ersten Elektrodenschichteinheit 18b ist zu dem Kontaktpunkt des ersten Kontaktelements 20b zu der ersten Elektrodenschichteinheit 18b wesentlich beabstandet. Das zweite Kontaktelement 22b ist auf einer dem ersten Kontaktelement 20b gegenüberliegenden Seite der ersten Elektrodenschichteinheit 18b angeordnet. Ein Teilbereich der ersten Elektrodenschichteinheit 18b ist zwischen dem ersten Kontaktelement 20b und dem zweiten Kontaktelement 22b angeordnet. Das erste Kontaktelement 20b und das zweite Kontaktelement 22b sind an gegenüberliegenden Punkten eines Gesamtumfangs der ersten Elektrodenschichteinheit 18b angeordnet. Des Weiteren weist der optische Blendschutzfilter 10b fünf weitere Kontaktelemente 24b, 26b, 28b, 30b, 32b auf. Die weiteren Kontaktelemente 24b, 26b, 28b, 30b, 32b sind ebenfalls zu einer elektrischen Kontaktierung der ersten Elektrodenschichteinheit 18b vorgesehen. Die weiteren Kontaktelemente 24b, 26b, 28b, 30b, 32b sind von dem ersten Kontaktelement 20b und dem zweiten Kontaktelement 22b sowie zueinander wesentlich beabstandet. Die Kontaktelemente 20b, 22b, 24b, 26b, 28b, 30b, 32b sind jeweils an einem Umfang der ersten Elektrodenschichteinheit 18b angeordnet. Die Kontaktelemente 20b, 22b, 24b, 26b, 28b, 30b, 32b sind entlang des Umfangs hintereinander angeordnet. Entlang des Umfangs benachbarte Kontaktelemente 20b, 22b, 24b, 26b, 28b, 30b, 32b sind jeweils wesentlich zueinander beabstandet. Elektrodenschichteinheit Die erste Elektrodenschichteinheit 18b ist an den Positionen der Kontaktelemente 20b, 22b, 24b, 26b, 28b, 30b, 32b durch eine nicht weiter sichtbare Dichtung der Flüssigkristallzelle 14b nach außen geführt, wo die Kontaktelemente 20b, 22b, 24b, 26b, 28b, 30b, 32b die Elektrodenschichteinheit 18b mit einem Flexprint kontaktieren. Die Kontaktelemente 20b, 22b, 24b, 26b, 28b, 30b, 32b bilden jeweils eine Kontaktierung der ersten Elektrodenschichteinheit 18b. Die Kontaktelemente 20b, 22b, 24b, 26b, 28b, 30b, 32b sind zu einer elektrischen Kontaktierung der ersten Elektrodenschichteinheit 18b in Umfangsrichtung um die erste Elektrodenschichteinheit 18b im Wesentlichen gleichmäßig verteilt angeordnet. Der optische Blendschutzfilter 10b weist ferner eine Kontakteinheit 56b auf. Die Kontakteinheit 56b weist die Kontaktelemente 20b, 22b, 24b, 26b, 28b, 30b, 32b auf. Ferner weist der optische Blendschutzfilter 10b sieben weitere Kontaktelemente 20b', 22b', 24b', 26b', 28b', 30b', 32b' auf, welche jeweils die zweite Elektrodenschichteinheit 18b' der Flüssigkristallzelle 14b elektrisch kontaktieren. Die zweite Elektrodenschichteinheit 18b' ist mit zu den Kontaktelementen 20b, 22b, 24b, 26b, 28b, 30b, 32b, welche die ersten Elektrodenschichteinheit 18b elektrisch kontaktieren, korrespondierenden Kontaktelementen 20b', 22b', 24b', 26b', 28b', 30b', 32b' verbunden. Die Kontaktelemente 20b', 22b', 24b', 26b', 28b', 30b', 32b', welche die zweite Elektrodenschichteinheit 18b' elektrisch kontaktieren, sind ebenfalls wesentlich zueinander beabstandet angeordnet. Die Kontaktelemente 20b', 22b', 24b', 26b', 28b', 30b', 32b', welche die zweite Elektrodenschichteinheit 18b' elektrisch kontaktieren, weisen gegenüber der zweiten Elektrodenschichteinheit 18b' die gleiche Anordnung auf wie Kontaktelemente 20b, 22b, 24b, 26b, 28b, 30b, 32b, welche die erste Elektrodenschichteinheit 18b elektrisch kontaktieren, gegenüber der ersten Elektrodenschichteinheit 18b. Die Kontakteinheit 56b weist die Kontaktelemente 20b', 22b', 24b', 26b', 28b', 30b', 32b' auf (Fig. 9, 10).

Des Weiteren weist der optische Blendschutzfilter 10b einen Nasenausschnitt 36b auf.

Figur 10 zeigt den optischen Blendschutzfilter 10b während eines Abdunklungsvorgangs zu einem Zeitpunkt t. Durch die multiple Kontaktierung der Elektrodenschichteinheiten 18b, 18b', ist es möglich, eine vorteilhaft hohe Schaltgeschwindigkeit der Flüssigkristallzelle 14b zu erreichen. Ferner kann eine vorteilhaft homogene Schaltgeschwindigkeitsverteilung der Flüssigkristallzelle 14b erreicht werden. Im Gegensatz zu einem nicht erfindungsgemäßen optischen Blendschutzfilter 66, bei welchem die Elektrodenschichteinheiten mit jeweils nur einem Kontaktelement 68 elektrisch kontaktiert werden, wie dies in Figur 8 dargestellt ist, kann zu dem selben Zeitpunkt t des Abdunklungsvorgangs mittels dem erfindungsgemäßen optischen Blendschutzfilter 10b eine wesentlich homogenere Schaltgeschwindigkeitsverteilung der Flüssigkristallzelle 14b sowie eine stärkere Abdunkelung der Flüssigkristallzelle 14b erreicht werden. Figur 10 zeigt schematisch einen Zustand der Abdunkelung der Flüssigkristallzelle 14b zu einem definierten Zeitpunkt t des Abdunklungsvorgangs.

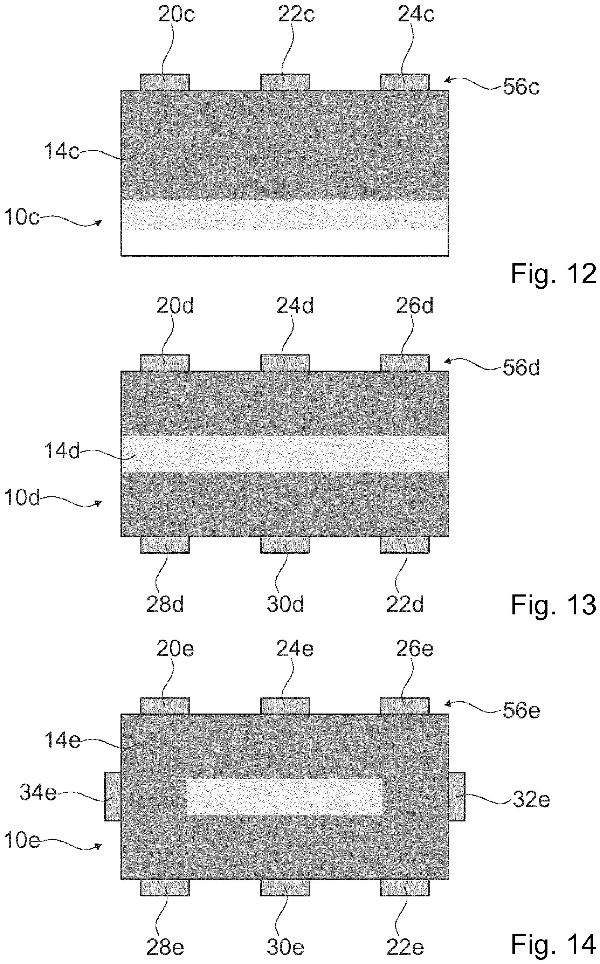

Die Figuren 12 bis 14 zeigen jeweils alternative optische Blendschutzfilter 10c, 10d, 10e mit einer Flüssigkristallzelle 14c, 14d, 14e und mit einer Kontakteinheit 56c, 56d, 56e. Die optischen Blendschutzfilter 10c, 10d, 10e weisen jeweils eine rechteckige Grundform auf. Die optischen Blendschutzfilter 10c, 10d, 10e weisen keinen Nasenausschnitt auf. Die Figuren 12 bis 14 zeigen die optischen Blendschutzfilter 10c, 10d, 10e während eines Abdunklungsvorgangs zu einem Zeitpunkt t. Durch die multiple Kontaktierung von Elektrodenschichteinheiten der Flüssigkristallzelle 14c, 14d, 14e ist es möglich, eine vorteilhaft hohe Schaltgeschwindigkeit der Flüssigkristallzelle 14c, 14d, 14e zu erreichen. Ferner kann eine vorteilhaft homogene Schaltgeschwindigkeitsverteilung der Flüssigkristallzelle 14c, 14d, 14e erreicht werden. Im Gegensatz zu einem nicht erfindungsgemäßen optischen Blendschutzfilter 66, bei welchem die Elektrodenschichteinheiten mit jeweils nur einem Kontaktelement 68 elektrisch kontaktiert werden, wie dies in Figur 11 dargestellt ist, kann zu dem selben Zeitpunkt t des Abdunklungsvorgangs mittels den erfindungsgemäßen optischen Blendschutzfiltern 10c, 10d, 10e eine wesentlich homogenere Schaltgeschwindigkeitsverteilung der Flüssigkristallzelle 14c, 14d, 14e sowie eine stärkere Abdunkelung der Flüssigkristallzelle 14c, 14d, 14e erreicht werden. Die Figuren 12 bis 14 zeigen schematisch einen Zustand der Abdunkelung der Flüssigkristallzelle 14c, 14d, 14e zu einem definierten Zeitpunkt t des Abdunklungsvorgangs.

Figur 12 zeigt einen optischen Blendschutzfilter 10c mit drei Kontaktelementen 20c, 22c, 24c auf. Die Kontaktelemente 20c, 22c, 24c sind nebeneinander angeordnet. Die Kontaktelemente 20c, 22c, 24c sind jeweils an einem Umfang der ersten Elektrodenschichteinheit angeordnet. Die erste Elektrodenschichteinheit 18c ist an den Positionen der Kontaktelemente 20c, 22c, 24c durch eine nicht weiter sichtbare Dichtung der Flüssigkristallzelle 14c nach außen geführt, wo die Kontaktelemente 20c, 22c, 24c die Elektrodenschichteinheit 18c mit einem Flexprint kontaktieren. Die Kontaktelemente 20c, 22c, 24c bilden jeweils eine Kontaktierung der ersten Elektrodenschicht. Die Kontakteinheit 56c weist die Kontaktelemente 20c, 22c, 24c auf. Ferner weist der optische Blendschutzfilter 10c drei weitere Kontaktelemente auf, welche jeweils die zweite Elektrodenschichteinheit der Flüssigkristallzelle 14c elektrisch kontaktieren. Die Kontaktelemente, welche die zweite Elektrodenschichteinheit elektrisch kontaktieren, weisen gegenüber der zweiten Elektrodenschichteinheit die gleiche Anordnung auf wie Kontaktelemente 20c, 22c, 24c, welche die erste Elektrodenschichteinheit elektrisch kontaktieren, gegenüber der ersten Elektrodenschicht.

Figur 13 zeigt einen optischen Blendschutzfilter 10d mit sechs Kontaktelementen 20d, 22d, 24d, 26d, 28d, 30d. Ein zweites Kontaktelement 22d ist auf einer einem ersten Kontaktelement 20d gegenüberliegenden Seite der ersten Elektrodenschichteinheit angeordnet. Die Kontaktelemente 20d, 22d, 24d, 26d, 28d, 30d sind jeweils an einem Umfang der ersten Elektrodenschichteinheit angeordnet. Die Kontaktelemente 20d, 22d, 24d, 26d, 28d, 30d sind entlang des Umfangs hintereinander angeordnet. Entlang des Umfangs benachbarte Kontaktelemente 20d, 22d, 24d, 26d, 28d, 30d sind jeweils wesentlich zueinander beabstandet. Die Kontaktelemente 20d, 22d, 24d, 26d, 28d, 30d bilden jeweils eine Kontaktierung der ersten Elektrodenschicht. Die Kontakteinheit 56d weist die Kontaktelemente 20d, 22d, 24d, 26d, 28d, 30d auf. Ferner weist der optische Blendschutzfilter 10d sechs weitere Kontaktelemente auf, welche jeweils die zweite Elektrodenschichteinheit der Flüssigkristallzelle 14d elektrisch kontaktieren. Die Kontaktelemente, welche die zweite Elektrodenschichteinheit elektrisch kontaktieren, weisen gegenüber der zweiten Elektrodenschichteinheit die gleiche Anordnung auf wie Kontaktelemente, 20d, 22d, 24d, 26d, 28d, 30d, welche die erste Elektrodenschichteinheit elektrisch kontaktieren, gegenüber der ersten Elektrodenschicht.

Figur 14 zeigt einen optischen Blendschutzfilter 10e mit acht Kontaktelementen 20e, 22e, 24e, 26e, 28e, 30e, 32e, 34e. Ein zweites Kontaktelement 22e ist auf einer einem ersten Kontaktelement 20e gegenüberliegenden Seite der ersten Elektrodenschichteinheit angeordnet. Die Kontaktelemente 20e, 22e, 24e, 26e, 28e, 30e, 32e, 34e sind jeweils an einem Umfang der ersten Elektrodenschichteinheit angeordnet. Die Kontaktelemente 20e, 22e, 24e, 26e, 28e, 30e, 32e, 34e sind entlang des Umfangs hintereinander angeordnet. Entlang des Umfangs benachbarte Kontaktelemente 20e, 22e, 24e, 26e, 28e, 30e, 32e, 34e sind jeweils wesentlich zueinander beabstandet. Die Kontaktelemente 20e, 22e, 24e, 26e, 28e, 30e, 32e, 34e bilden jeweils eine Kontaktierung der ersten Elektrodenschicht. Die Kontaktelemente 20e, 22e, 24e, 26e, 28e, 30e, 32e, 34e sind zu einer elektrischen Kontaktierung der ersten Elektrodenschichteinheit in Umfangsrichtung um die erste Elektrodenschichteinheit im Wesentlichen gleichmäßig verteilt angeordnet. Die Kontakteinheit 56e weist die Kontaktelemente 20e, 22e, 24e, 26e, 28e, 30e, 32e, 34e auf. Ferner weist der optische Blendschutzfilter 10e acht weitere Kontaktelemente auf, welche jeweils die zweite Elektrodenschichteinheit der Flüssigkristallzelle 14e elektrisch kontaktieren. Die Kontaktelemente, welche die zweite Elektrodenschichteinheit elektrisch kontaktieren, weisen gegenüber der zweiten Elektrodenschichteinheit die gleiche Anordnung auf wie Kontaktelemente 20e, 22e, 24e, 26e, 28e, 30e, 32e, 34e, welche die erste Elektrodenschichteinheit elektrisch kontaktieren, gegenüber der ersten Elektrodenschicht. Eine Anordnung und Anzahl der Kontaktelemente 20e, 22e, 24e, 26e, 28e, 30e, 32e, 34e ist hierbei lediglich beispielhaft zu verstehen. Grundsätzlich wäre auch eine andere, einem Fachmann als sinnvoll erscheinende Anzahl und/oder Anordnung der Kontaktelemente 20e, 22e, 24e, 26e, 28e, 30e, 32e, 34e denkbar.

## Patentansprüche

1. Optischer Blendschutzfilter für eine Blendschutzvorrichtung (12a), mit zumindest einer Flüssigkristallzelle (14a; 14b; 14c; 14d; 14e), welche zumindest eine Flüssigkristallschicht (16a; 16b) und zumindest eine erste Elektrodenschichteinheit (18a; 18b) zu einer Ausrichtung von Kristallmolekülen der zumindest einen Flüssigkristallschicht (16a, 16b) aufweist, und mit zumindest einem ersten Kontaktelement (20a; 20b; 20c; 20d; 20e) zu einer elektrischen Kontaktierung der zumindest einen ersten Elektrodenschichteinheit (18a; 18b),
**gekennzeichnet durch**
zumindest ein zweites Kontaktelement (22a; 22b; 22c; 22d; 22e) zu einer elektrischen Kontaktierung der zumindest einen ersten Elektrodenschichteinheit (18a; 18b), welches wesentlich von dem ersten Kontaktelement (20a; 20b; 20c; 20d; 20e) beabstandet ist.

2. Optischer Blendschutzfilter nach Anspruch 1,
**dadurch gekennzeichnet, dass**
das zumindest eine zweite Kontaktelement (22b; 22d; 22e) auf einer dem zumindest einen ersten Kontaktelement (20b; 20d; 20e) gegenüberliegenden Seite der ersten Elektrodenschichteinheit (18b) angeordnet ist.

3. Optischer Blendschutzfilter nach einem der vorhergehenden Ansprüche,
**gekennzeichnet durch**
zumindest ein weiteres Kontaktelement (24a; 24b, 26b, 28b, 30b, 32b; 24c; 24d, 26d, 28d, 30d; 24e, 26e, 28e, 30e, 32e, 34e) zu einer elektrischen Kontaktierung der zumindest einen ersten Elektrodenschichteinheit (18a; 18b), welche wesentlich von dem ersten Kontaktelement (20a; 20b; 20c; 20d; 20e) und dem zweiten Kontaktelement (22a; 22b; 22c; 22d; 22e) beabstandet ist.

4. Optischer Blendschutzfilter nach einem der vorhergehenden Ansprüche,
**dadurch gekennzeichnet, dass**
die Kontaktelemente (20b, 22b, 24b, 26b, 28b, 30b, 32b; 20e, 22e, 24e, 26e, 28e, 30e, 32e, 34e) zu einer elektrischen Kontaktierung der zumindest einen ersten Elektrodenschichteinheit (18b) in Umfangsrichtung um die erste Elektrodenschichteinheit (18b) zumindest im Wesentlichen gleichmäßig verteilt sind.

5. Optischer Blendschutzfilter nach einem der vorhergehenden Ansprüche,
**gekennzeichnet durch**
einen Nasenausschnitt (36a; 36b), welcher zu einer zumindest teilweisen Aufnahme einer Nase eines Benutzers (38a) vorgesehen ist.

6. Optischer Blendschutzfilter nach einem der vorhergehenden Ansprüche,
**gekennzeichnet durch**
zumindest zwei weitere Kontaktelemente (20a', 22a', 24a'; 20b', 22b', 24b', 26b', 28b', 30b', 32b'), welche jeweils eine zweite Elektrodenschichteinheit (18a'; 18b') der zumindest einen Flüssigkristallzelle (14a; 14b; 14c; 14d; 14e) elektrisch kontaktieren.

7. Blendschutzvorrichtung mit zumindest einem optischen Blendschutzfilter (10a; 10b; 10c; 10d; 10e) nach einem der vorhergehenden Ansprüche und mit zumindest einer Steuer- und/oder Regeleinheit (40a), welche dazu vorgesehen ist, abhängig von einem erfassten Arbeitszustand und/oder einer Lichteinstrahlung eine Durchlässigkeit des optischen Blendschutzfilters (10a; 10b; 10c; 10d; 10e) zu steuern und/oder zu regeln.

8. Blendschutzvorrichtung nach Anspruch 7,
**dadurch gekennzeichnet, dass**
die zumindest eine Steuer- und/oder Regeleinheit (40a) den optischen Blendschutzfilter (10a; 10b; 10c; 10d; 10e) zumindest im Wesentlichen gleichzeitig über das zumindest eine erste Kontaktelement (20a, 20a'; 20b, 20b'; 20c, 20c'; 20d, 20d'; 20e, 20e') und das eine zweite Kontaktelement (22a, 22a'; 22b, 22b'; 22c, 22c'; 22d, 22d'; 22e, 22e') ansteuert.

9. Blendschutzvorrichtung nach Anspruch 7 oder 8,
**gekennzeichnet durch**
zumindest eine Schildeinheit (42a), in welcher der zumindest eine optische Blendschutzfilter (10a; 10b; 10c; 10d; 10e) fest aufgenommen ist.

## Claims

1. Optical glare protection filter for a glare protection device (12a), with at least one liquid-crystal cell (14a; 14b; 14c; 14d; 14e) comprising at least one liquid-crystal layer (16a; 16b) and at least one first electrode layer unit (18a; 18b) for an alignment of crystal molecules of the at least one liquid-crystal layer (16a; 16b), and with at least one first contact element (20a; 20b; 20c; 20d; 20e) for electrically contacting the at least one first electrode layer unit (18a; 18b),
**characterised by** at least one second contact element (22a; 22b; 22c; 22d; 22e) for electrically contacting the at least one first electrode layer unit (18a; 18b), which is substantially spaced apart from the first contact element (20a; 20b, 20c; 20d; 20e).

2. Optical glare protection filter according to claim 1,
**characterised in that** the at least one second contact element (22b; 22d; 22e) is arranged on a side of the first electrode layer unit (18b) that is situated opposite the at least one first contact element (20b; 20d; 20e).

3. Optical glare protection filter according to one of the preceding claims,
**characterised by** at least one further contact element (24a; 24b, 26b, 28b, 30b, 32b; 24c; 24d, 26d, 28d, 30d; 24e, 26e, 28e, 30e, 32e, 34e) for electrically contacting the at least one first electrode layer unit (18a; 18b), which is substantially spaced apart from the first contact element (20a; 20b; 20c; 20d; 20e) and the second contact element (22a; 22b; 22c; 22d; 22e).

4. Optical glare protection filter according to one of the preceding claims,
**characterised in that** for the purpose of electrically contacting the at least one first electrode layer unit (18b), the contact elements (20b, 22b, 24b, 26b, 28b, 30b, 32b; 20e, 22e, 24e, 26e, 28e, 30e, 32e, 34e) are distributed evenly in a circumferential direction around the first electrode layer unit (18b).

5. Optical glare protection filter according to one of the preceding claims,
**characterised by** a nose cut-out (36a; 36b), which is configured to at least partially accommodate a user's (38a) nose.

6. Optical glare protection filter according to one of the preceding claims,
**characterised by** at least two further contact elements (20a', 22a', 24a'; 20b', 22b', 24b', 26b', 28b', 30b', 32b'), each of which electrically contacts a second electrode layer unit (18a; 18b') of the at least one liquid-crystal cell (14a; 14b; 14c; 14d; 14e).

7. Glare protection device with at least one optical glare protection filter (10a; 10b; 10c; 10d; 10e) according to one of the preceding claims, and with at least one control and/or regulation unit (40a) that is configured to control and/or regulate a transmittance of the optical glare protection filter (10a; 10b; 10c; 10d; 10e) depending on a captured work state and/or on a light irradiation.

8. Glare protection device according to claim 7,
**characterised in that** the at least one control and/or regulation unit (40a) actuates the optical glare protection filter (10a; 10b; 10c; 10d; 10e) at least substantially simultaneously via the at least one first contact element (20a, 20a'; 20b, 20b'; 20c, 20c'; 20d, 20d'; 20e, 20e') and the one second contact element (22a, 22a'; 22b, 22b'; 22c, 22c'; 22d, 22d'; 22e, 22e').

9. Glare protection device according to claim 7 or 8,
**characterised by** at least one shield unit (42a), in which the at least one optical glare protection filter (10a; 10b; 10c; 10d; 10e) is fixedly accommodated.

## Revendications

1. Filtre optique anti-éblouissement pour un dispositif anti-éblouissement (12a)
avec au moins une cellule à cristaux liquides (14a ; 14b ; 14c ; 14d ; 14e) comprenant au moins une couche à cristaux liquides (16a ; 16b) et au moins une première unité de couche-électrode (18a ; 18b) pour un alignement de molécules cristallins de l'au moins une couche à cristaux liquides (16a ; 16b),
et avec au moins un premier élément de contact (20a ; 20b ; 20c ; 20d ; 20e) pour une mise en contact électrique avec l'au moins une première unité de couche-électrode (18a ; 18b),
**caractérisé par** au moins un deuxième élément de contact (22a ; 22b ; 22c ; 22d ; 22e) pour une mise en contact électrique avec l'au moins une première unité de couche-électrode (18a ; 18b), ledit deuxième élément de contact (22a ; 22b, 22c ; 22d ; 22e) étant sensiblement écarté du premier élément de contact (20a ; 20b ; 20c ; 20d ; 20e).

2. Filtre optique anti-éblouissement selon la revendication 1,
**caractérisé en ce que** l'au moins un deuxième élément de contact (22b ; 22d ; 22e) est disposé sur un côté de la première unité de couche-électrode (18b) opposé à l'au moins un premier élément de contact (20b ; 20d ; 20e).

3. Filtre optique anti-éblouissement selon l'une quelconque des revendications précédentes,
**caractérisé par** au moins un élément de contact de plus (24a ; 24b, 26b, 28b, 30b, 32b ; 24c ; 24d, 26d, 28d, 30d ; 24e, 26e, 28e, 30e, 32e, 34e) pour une mise en contact électrique avec l'au moins une première unité de couche-électrode (18a ; 18b), qui est sensiblement écarté du premier élément de contact (20a ; 20b ; 20c ; 20d ; 20e) et du deuxième élément de contact (22a ; 22b ; 22c ; 22d ; 22e).

4. Filtre optique anti-éblouissement selon l'une quelconque des revendications précédentes,
**caractérisé en ce que** pour une mise en contact avec l'au moins une première unité de couche-électrode (18b) les éléments de contact (20b, 22b, 24b, 26b, 28b, 30b, 32b ; 20e, 22e, 24e, 26e, 28e, 30e, 32e, 34e) sont distribués autour de la première unité de couche-électrode (18b) en direction circonférentielle d'une façon au moins sensiblement régulière.

5. Filtre optique anti-éblouissement selon l'une quelconque des revendications précédentes,
**caractérisé par** une encoche-nez (36a ; 36b) prévue pour au moins partiellement recevoir le nez d'un utilisateur (38a).

6. Filtre optique anti-éblouissement selon l'une quelconque des revendications précédentes,
**caractérisé par** au moins deux éléments de contact de plus (20a', 22a', 24a' ; 20b', 22b', 24b', 26b', 28b', 30b', 32b'), chacun desquels électriquement contactant une deuxième unité de couche-électrode (18a' ; 18b') de l'au moins une cellule à cristaux liquides (14a ; 14b ; 14c ; 14d ; 14e).

7. Dispositif anti-éblouissement avec au moins un filtre optique anti-éblouissement (10a ; 10b, 10c ; 10d ; 10e) selon l'une quelconque des revendications précédentes et avec au moins une unité de commande et/ou régulation (40a), qui est prévue pour commander et/ou réguler une transmittance du filtre optique anti-éblouissement (10a ; 10b ; 10c ; 10d ; 10e) en fonction d'un état opératif détecté et/ou d'une irradiation lumineuse.

8. Dispositif anti-éblouissement selon la revendication 7,
**caractérisé en ce que** l'au moins une unité de commande et/ou régulation (40a) actionne le filtre optique anti-éblouissement (10a ; 10b ; 10c ; 10d ; 10e) au moins sensiblement simultanément via l'au moins un premier élément de contact (20a, 20a' ; 20b, 20b' ; 20c, 20c' ; 20d, 20d' ; 20e, 20e') et l'un deuxième élément de contact (22a, 22a' ; 22b, 22b' ; 22c, 22c ; 22d, 22d' ; 22e, 22e').

9. Dispositif anti-éblouissement selon l'une quelconque des revendications 7 ou 8,
**caractérisé par** au moins une unité écran (42a), dans laquelle l'au moins un filtre optique anti-éblouissement (10a ; 10b ; 10c ; 10d ; 10e) est fixement reçu.
